# EUROPEAN PATENT SPECIFICATION

(11) **EP 3 841 954 B1**
(45) Date of publication and mention of the grant of the patent: **12.11.2025**
(21) Application number: 19851797.1
(22) Date of filing: 08.08.2019
(51) Int. Cl.: A61B 1/045, G06T 19/20, A61B 1/00

(54) **MEDICAL IMAGE PROCESSING SYSTEM**
MEDIZINISCHES BILDVERARBEITUNGSSYSTEM
SYSTÈME DE TRAITEMENT D'IMAGE MÉDICALE

(30) Priority: 20.08.2018 JP 2018154120
(43) Date of publication of application: 30.06.2021
(73) Proprietor: FUJIFILM Corporation, Tokyo 106-8620 (JP)
(72) Inventor: KARINO, Takatoshi, Ashigarakami-gun Kanagawa 258-8538 (JP)
(74) Representative: Dehns Germany Partnerschaft mbB
(86) International application number: PCT/JP2019/031432
(87) International publication number: WO 2020/039968

(56) References cited:
- EP-A1- 2 901 935
- WO-A1-2017/081976
- WO-A1-2018/003503
- JP-A- 2011 115 264
- US-A1- 2011 254 937

## Description

### BACKGROUND OF THE INVENTION

### 1. Field of the Invention

The present invention relates to a medical image processing system using an analysis result of a medical image.

### 2. Description of the Related Art

In the current medical field, medical image processing systems using medical images, such as an endoscope system including a light source device, an endoscope, and a processor device, are widely used. In recent years, diagnostic information regarding the state of a disease has been acquired by extracting a region of interest containing a potential lesion portion from a medical image and performing image analysis on the extracted region of interest.

For example, in WO2017/073338A, a region of interest in a medical image is detected based on a specific feature value for the medical image. In response to the detection of the region of interest, a notification image notifying that a feature region has been detected is displayed in an outer screen, and a marker image is added to the feature region in an inner screen. US 2011/0254937 A1 discloses an image processing device. The device includes a special light image acquisition section that acquires an image including an object image that includes information in a specific wavelength band as a special light image, an attention area detection section that detects an attention area based on a feature quantity of each pixel of the special light image, a disappearance determination section that determines whether or not the attention area has disappeared from a display target area based on a detection result for the attention area, the display target area being an area displayed on an output section, a guide information generation section that generates guide information about the attention area that has disappeared based on a determination result of the disappearance determination section, and an output control section that controls output of information including the guide information generated by the guide information generation section.
EP 2 901 935 A1 discloses that a structure extracting unit extracts a structure from a three-dimensional medical image, and a view point determining unit determines a view point position and a direction of line of sight of a virtual endoscopic image. An image generating unit calculates a distance between a view point position and the extracted structure, determines a display attribute of the extracted structure based on the distance and a plurality of different display attributes that correspond to different distances from the view point position and are defined for each of the structures, and generates, from the three-dimensional medical image, a virtual endoscopic image containing the structure having the determined display attribute. A display control unit displays the thus generated virtual endoscopic image on a display.

### SUMMARY OF THE INVENTION

As in WO2017/073338A, in the case of detecting a region of interest, detection accuracy may be low depending on the detection position of the region of interest. For example, regarding an endoscopic image among medical images, a peripheral region (such as an end portion) of the endoscopic image contains image characteristics provided by a lens (typically, a lens having a wide angle of view is used), irradiation unevenness of illumination light, and so on. The detection accuracy of the region of interest in the peripheral region of the endoscopic image may be thus lower than that in any other region such as a center region thereof. Accordingly, in a case where the detection accuracy of the region of interest is different between the center region and the peripheral region, if a detection result in the center region and a detection result in the peripheral region are displayed in the same manner, the doctor may not be able to make an accurate diagnosis.

In addition, depending on the detection position of the region of interest, the user is likely to fail to direct attention to the region of interest. For example, when an endoscope is inserted, attention is directed to the center region corresponding to the direction of movement (direction of retreat), whereas attention is not directed to the peripheral region around the center region.

It is an object of the present invention to provide a medical image processing system capable of providing a notification of, when detecting a region of interest based on a medical image, a detection result of the region of interest in accordance with the detection accuracy and preventing the detection result of the region of interest from being overlooked using the detection position of the region of interest.

A medical image processing system of the present invention includes a medical image acquisition unit, a region-of-interest detection unit, and a display control unit. The medical image acquisition unit acquires a medical image obtained by imaging of an observation target. The region-of-interest detection unit detects a region of interest from the medical image. The display control unit displays a detection result of the region of interest on a display unit using a display style that differs depending on at least the detection position of the region of interest. The region of interest is preferably a target desired as a region of interest by a user, examples of which include a lesion portion, a scar after a treatment, a scar from an operation, a bleeding location, a benign tumor portion, an inflammation portion, a marking portion, and a biopsy-performing portion.

Preferably the medical image is divided into a plurality of regions, and the display style of the detection result of the region of interest is changed according to which of the plurality of regions the detection position of the region of interest is included in. Preferably, a display style of the detection result of the region of interest when the detection position of the region of interest is included in a first region of the medical image is different from a display style of the detection result of the region of interest when the detection position of the region of interest is included in a second region of the medical image different from the first region. Preferably, the first region is a center region of the medical image, and the second region is a peripheral region around the center region of the medical image.

A display style of the detection result of the region of interest when a specific distance indicating a distance between a predetermined specific position and the detection position of the region of interest falls within a specific range is different from a display style of the detection result of the region of interest when the specific distance falls outside the specific range. The specific position is included in a center region of the medical image.

Preferably, when the detection result of the region of interest is represented by a specific geometric shape, a line thickness of the specific geometric shape differs depending on the detection position of the region of interest. Preferably, when the detection result of the region of interest is represented by a specific geometric shape, a size of the specific geometric shape differs depending on the detection position of the region of interest. Preferably, when the detection result of the region of interest is represented by a specific geometric shape, a display time of the specific geometric shape differs depending on the detection position of the region of interest. Preferably, when the detection result of the region of interest is represented by a specific geometric shape, a transparency of the specific geometric shape differs depending on the detection position of the region of interest. Preferably, when the detection result of the region of interest is represented by a specific color region, a color of the specific color region differs depending on the detection position of the region of interest. Preferably, when the detection result of the region of interest is represented by a specific color region, a display time of the specific color region differs depending on the detection position of the region of interest.

According to the present invention, it is possible to provide a notification of, when detecting a region of interest based on a medical image, a detection result of the region of interest in accordance with the detection accuracy and to prevent the detection result of the region of interest from being overlooked using the detection position of the region of interest.

### BRIEF DESCRIPTION OF THE DRAWINGS

Fig. 1 is a block diagram illustrating an image processing system, an endoscope system, and so on;
Fig. 2 is a block diagram illustrating the endoscope system;
Fig. 3 is a block diagram illustrating the functions of a medical image analysis processing unit;
Fig. 4 is an image diagram illustrating a center region and a peripheral region in a medical image;
Fig. 5 is an image diagram of a medical image indicating that the thickness of a bounding box differs depending on the detection position of a region of interest;
Fig. 6 is an image diagram of a medical image different from that in Fig. 5 and is an image diagram of a medical image indicating that the thickness of a bounding box differs depending on the detection position of the region of interest;
Fig. 7 is an image diagram of a medical image indicating that the size of a bounding box differs depending on the detection position of the region of interest;
Fig. 8 is an image diagram of a medical image different from that in Fig. 7 and is an image diagram of a medical image indicating that the size of a bounding box differs depending on the detection position of the region of interest;
Fig. 9 is an explanatory diagram illustrating that the display time of a bounding box differs depending on the detection position of the region of interest;
Fig. 10 is an explanatory diagram different from that in Fig. 9 and is an explanatory diagram illustrating that the display time of a bounding box differs depending on the detection position of the region of interest;
Fig. 11 is an image diagram of a medical image indicating that the transparency of a bounding box differs depending on the detection position of the region of interest;
Fig. 12 is an image diagram of a medical image different from that in Fig. 11 and is an image diagram of a medical image indicating that the transparency of a bounding box differs depending on the detection position of the region of interest.
Fig. 13 is an image diagram of a medical image indicating that the color of a specific color region differs depending on the detection position of the region of interest;
Fig. 14 is an explanatory diagram illustrating that the display time of a specific color region differs depending on the detection position of the region of interest;
Fig. 15 is an explanatory diagram different from that in Fig. 14 and is an explanatory diagram illustrating that the display time of a specific color region differs depending on the detection position of the region of interest;
Fig. 16 is an image diagram of a medical image indicating a specific position SP, a detection position DP of the region of interest, and a specific distance L;
Fig. 17 illustrates a diagnosis support apparatus including the image processing system; and
Fig. 18 illustrates a medical operation support apparatus including the image processing system.

### DESCRIPTION OF THE PREFERRED EMBODIMENTS

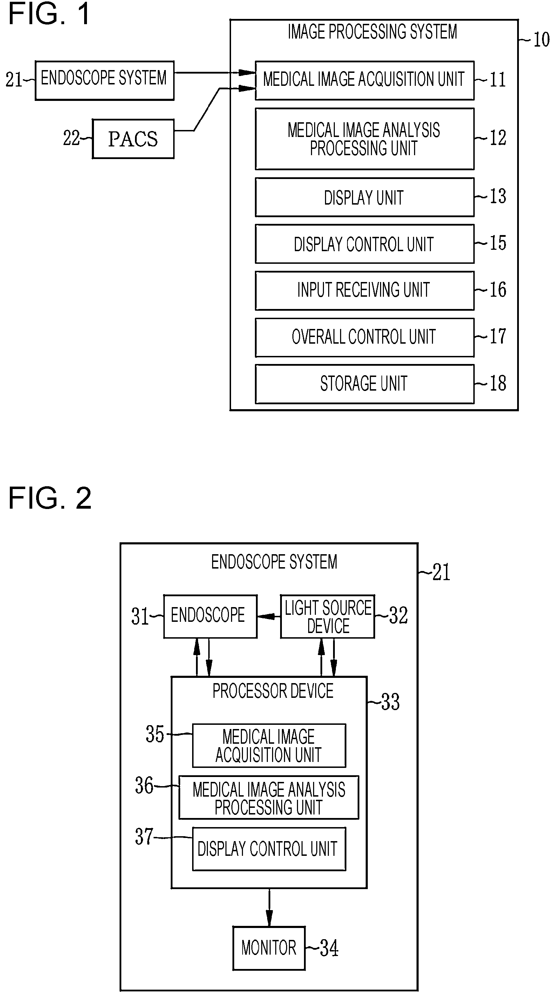

As illustrated in Fig. 1, an image processing system 10 includes a medical image acquisition unit 11, a medical image analysis processing unit 12, a display unit 13, a display control unit 15, an input receiving unit 16, an overall control unit 17, and a storage unit 18.

The medical image acquisition unit 11 acquires a medical image including a photographic subject image directly from an endoscope system 21 or the like that is a medical apparatus, or via a management system such as a PACS (Picture Archiving and Communication System) 22 or any other information system. The medical image is a still image or a moving image (so-called examination moving image). When the medical image is a moving image, the medical image acquisition unit 11 can acquire, as still images, frame images constituting the moving image after an examination. When the medical image is a moving image, furthermore, displaying the medical image includes displaying a still image of one representative frame constituting the moving image, and reproducing the moving image one or a plurality of times. The medical image acquired by the medical image acquisition unit 11 includes an image captured by a doctor using a medical apparatus such as the endoscope system 21, and an image automatically captured by the medical apparatus such as the endoscope system 21 regardless of an image-capturing instruction given by the doctor. In this embodiment, since the image processing system 10 and the endoscope system 21 perform image processing using a medical image, both the image processing system 10 and the endoscope system 21 correspond to a medical image processing system.

When a plurality of medical images can be acquired, the medical image acquisition unit 11 can selectively acquire one or a plurality of medical images among these medical images. Further, the medical image acquisition unit 11 can acquire a plurality of medical images acquired in a plurality of different examinations. For example, the medical image acquisition unit 11 can acquire either or both of a medical image acquired in an examination performed in the past and a medical image acquired in the latest examination. That is, the medical image acquisition unit 11 can arbitrarily acquire a medical image.

In this embodiment, a plurality of medical images including photographic subject images are acquired. More specifically, in a case where a medical image captured in a single specific examination is acquired and there is a plurality of medical images captured in a single specific examination, a plurality of medical images are acquired from among the series of medical images. In this embodiment, furthermore, the image processing system 10 is connected to the endoscope system 21 to acquire a medical image from the endoscope system 21. That is, in this embodiment, a medical image is an endoscopic image.

The display unit 13 is a display that displays the medical image acquired by the medical image acquisition unit 11 and an analysis result obtained by the medical image analysis processing unit 12. A monitor or display included in a device to which the image processing system 10 is connected can be shared and used as the display unit 13 of the image processing system 10. The display control unit 15 controls a display style of the medical image and the analysis result on the display unit 13.

The input receiving unit 16 accepts an input from a mouse, a keyboard, or any other operating device connected to the image processing system 10. The operation of the units of the image processing system 10 can be controlled using these operating devices.

The overall control unit 17 performs overall control of the operation of the units of the image processing system 10. When the input receiving unit 16 receives an operation input using an operating device, the overall control unit 17 controls the units of the image processing system 10 in accordance with the operation input.

The storage unit 18 stores a still image or the like of a medical image in a storage device (not illustrated) such as a memory included in the image processing system 10 or in a storage device (not illustrated) included in the medical apparatus such as the endoscope system 21 or the PACS 22.

As illustrated in Fig. 2, in this embodiment, the endoscope system 21 to which the image processing system 10 is connected includes an endoscope 31 that captures an image of a photographic subject irradiated with at least one of light in the white wavelength range or light in a specific wavelength range to acquire an image, a light source device 32 that irradiates the inside of the photographic subject with illumination light via the endoscope 31, a processor device 33, and a monitor 34 that displays a endoscopic image or the like captured using the endoscope 31. The light in the specific wavelength range to be used as illumination light by the endoscope 31 is, for example, light in a shorter wavelength range than the green wavelength range and is, in particular, light in the blue range or violet range in the visible range.

The processor device 33 includes a medical image acquisition unit 35, a medical image analysis processing unit 36, and a display control unit 37. The medical image acquisition unit 35 acquires the medical image output from the endoscope 31. The medical image analysis processing unit 36 performs analysis processing on the medical image acquired by the medical image acquisition unit 35. The content of the processing performed by the medical image analysis processing unit 36 is similar to the content of the processing performed by the medical image analysis processing unit 12 of the image processing system 10. The display control unit 37 displays the medical image obtained by the medical image analysis processing unit 36 on the monitor 34. The processor device 33 is connected to the image processing system 10. The medical image acquisition unit 35 is similar to the medical image acquisition unit 11, the medical image analysis processing unit 36 is similar to the medical image analysis processing unit 12, and the display control unit 37 is similar to the display control unit 15.

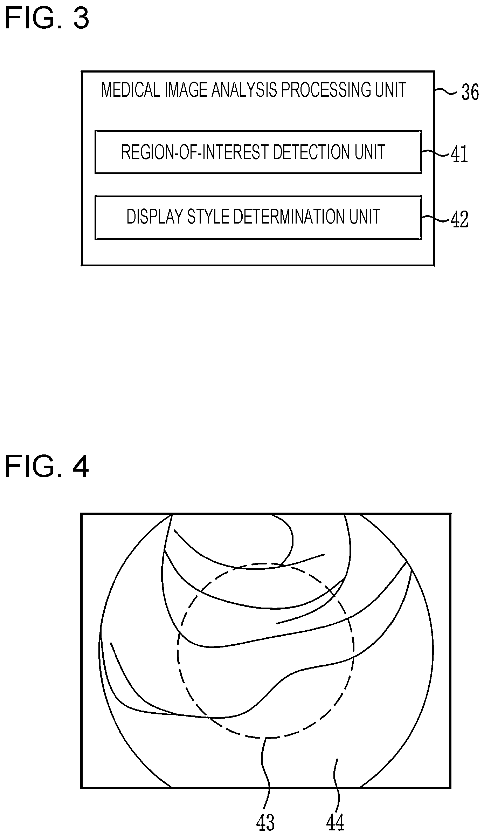

The medical image analysis processing unit 36 performs analysis processing using the medical image acquired by the medical image acquisition unit 35. As illustrated in Fig. 3, the medical image analysis processing unit 36 includes a region-of-interest detection unit 41 and a display style determination unit 42. The region-of-interest detection unit 41 performs region-of-interest detection processing for detecting a region of interest from the medical image. Examples of the region-of-interest detection processing include NN (Neural Network), CNN (Convolutional Neural Network), AdaBoost, and random forest. Alternatively, the region-of-interest detection processing may involve detecting a region of interest on the basis of a feature value obtained as color information of the medical image, the gradient of pixel values, or the like. The gradient of pixel values or the like changes according to, for example, the shape of the photographic subject (such as generalized ups and downs or localized depression or elevation in a mucous membrane), color (color such as from inflammation, bleeding, redness, or whitening caused by atrophy), tissue characteristics (such as the thickness, depth, or density of blood vessels, or a combination thereof), structural characteristics (such as pit pattern), or the like.

The region of interest detected by the region-of-interest detection unit 41 is a region including, for example, a lesion portion such as a cancer, a scar after a treatment, a scar from an operation, a bleeding location, a benign tumor portion, an inflammation portion (including, in addition to so-called inflammations, a portion with a change such as bleeding or atrophy), an ablation mark by heating, a marking portion marked by coloring with a coloring agent, a fluorescent agent, or the like, or a biopsy-performing portion subjected to a biopsy. That is, a region including a lesion, a region of a potential lesion, a region subjected to some treatment such as a biopsy, a treatment tool such as a clip or forceps, a region requiring detailed observation regardless of the possibility of a lesion, such as a dark region (a region where observation light is difficult to reach because of the back of the fold or the back of the lumen), or the like can be a region of interest. In the endoscope system 21, the region-of-interest detection unit 41 detects, as a region of interest, a region including at least one of a lesion portion, a scar after a treatment, a scar from an operation, a bleeding location, a benign tumor portion, an inflammation portion, a marking portion, or a biopsy-performing portion.

The display style determination unit 42 determines the display style of the detection result of the region of interest on the basis of the detection position of the region of interest. The display control unit 37 displays the detection result of the region of interest in the medical image on the monitor 34 in accordance with the display style of the detection result of the region of interest. Due to the image characteristics provided by a lens (typically, a lens having a wide angle of view is used) used in the endoscope 31, irradiation unevenness of illumination light, and so on, the detection accuracy differs depending on the detection position of the region of interest detected from the medical image. Thus, it is preferable to change the display style of the detection result of the region of interest. In consideration of a region where the user is likely to overlook the region of interest and a region where the user is less likely to overlook the region of interest, it is preferable to change the display style of the detection result of the region of interest according to the detection position of the region of interest.

For example, it is preferable that the display style of the detection result of the region of interest when, as illustrated in Fig. 4, the detection position of the region of interest is included in a center region 43 (first region) of the medical image be different from the display style of the detection result of the region of interest when the detection position of the region of interest is included in a peripheral region 44 (second region) of the medical image. For example, when the detection result of the region of interest is represented by a specific geometric shape, the line thickness of the specific geometric shape used to provide a notification of the region of interest detected in the center region 43 is made different from the line thickness of the specific geometric shape used to provide a notification of the region of interest detected in the peripheral region 44. The geometry (for example, star) of the specific geometric shape used to provide a notification of the region of interest detected in the center region 43 and the geometry (for example, circle) of the specific geometric shape used to provide a notification of the region of interest detected in the peripheral region 44 may be made different.

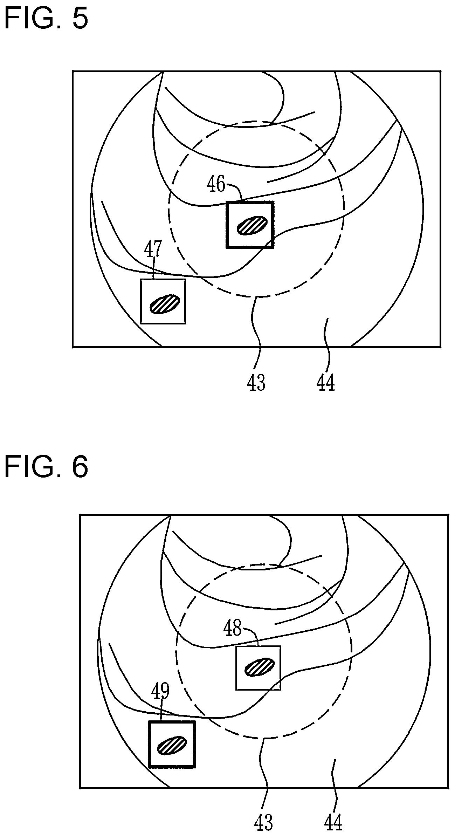

When a bounding box is used as the specific geometric shape and the detection accuracy of the region of interest is represented by the thickness of bounding box, as illustrated in Fig. 5, it is preferable that the frame thickness of a bounding box 46 used to provide a notification of the region of interest detected in the center region 43 be larger than the frame thickness of a bounding box 47 used to provide a notification of the region of interest detected in the peripheral region 44. This allows the user to grasp the detection accuracy using the frame thickness of the bounding boxes 46 and 47. That is, the user is able to grasp that the detection accuracy of the region of interest detected in the center region 43 is high, whereas the detection accuracy of the region of interest detected in the peripheral region 44 is low.

On the other hand, when a bounding box is used as the specific geometric shape and the likelihood of oversight of the region of interest is represented by the frame thickness of the bounding box, as illustrated in Fig. 6, it is preferable that the frame thickness of a bounding box 48 used to provide a notification of the region of interest detected in the center region 43 be smaller than the frame thickness of a bounding box 49 used to provide a notification of the region of interest detected in the peripheral region 44. This can prevent the region of interest detected in the peripheral region 44 from being overlooked since the bounding box 49 is high in visibility. In contrast, the region of interest detected in the center region 43 can be made less noticeable since the bounding box 48 is low in visibility.

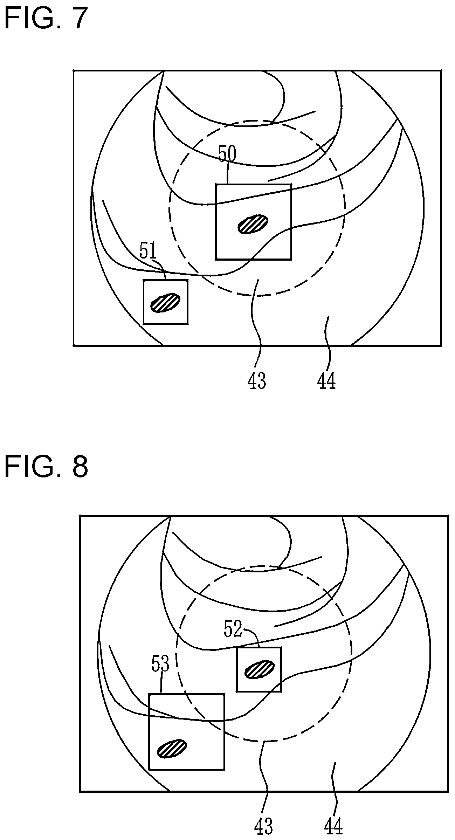

Further, when the detection result of the region of interest is represented by a specific geometric shape, the size of the specific geometric shape used to provide a notification of the region of interest detected in the center region 43 is made different from the size of the specific geometric shape used to provide a notification of the region of interest detected in the peripheral region 44. In this case, as illustrated in Fig. 7, when a bounding box is used as the specific geometric shape and the detection accuracy of the region of interest is represented by the size of the bounding box, it is preferable that the size of a bounding box 50 used to provide a notification of the region of interest detected in the center region 43 be larger than the size of a bounding box 51 used to provide a notification of the region of interest detected in the peripheral region 44. This allows the user to grasp the detection accuracy using the size of bounding boxes 50 and 51. That is, the user is able to grasp that the detection accuracy of the region of interest detected in the center region 43 is high, whereas the detection accuracy of the region of interest detected in the peripheral region 44 is low.

On the other hand, when a bounding box is used as the specific geometric shape and the likelihood of oversight of the region of interest is represented by the size of the bounding box, as illustrated in Fig. 8, it is preferable that the size of a bounding box 52 used to provide a notification of the region of interest detected in the center region 43 be smaller than the size of a bounding box 53 used to provide a notification of the region of interest detected in the peripheral region 44. This can prevent the region of interest detected in the peripheral region 44 from being overlooked since the bounding box 53 is displayed large. In contrast, the region of interest detected in the center region 43 can be made less noticeable since the bounding box 52 is displayed small.

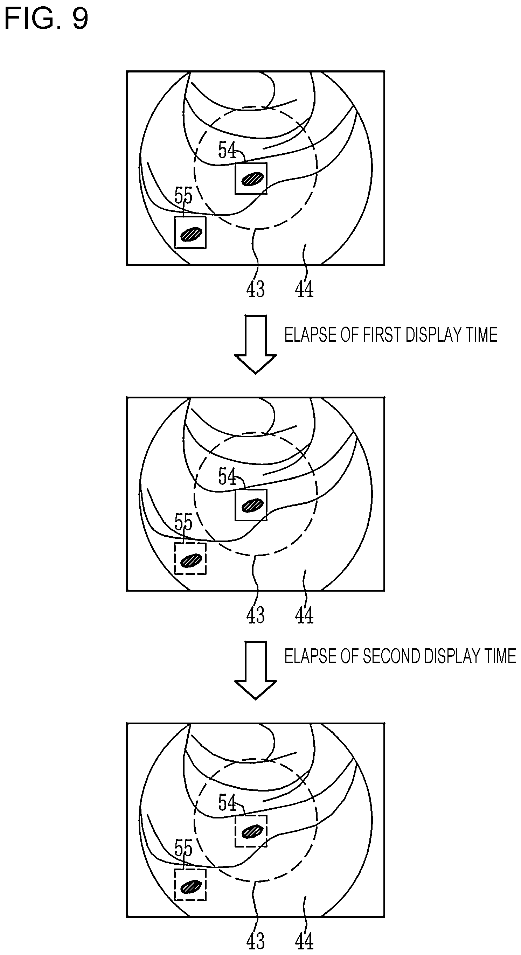

Further, when the detection result of the region of interest is represented by a specific geometric shape, the time (display time) during which the specific geometric shape used to provide a notification of the region of interest detected in the center region 43 continues to be displayed on the monitor 34 is made different from the display time of the specific geometric shape used to provide a notification of the region of interest detected in the peripheral region 44. In this case, as illustrated in Fig. 9, when a bounding box is used as the specific geometric shape and the detection accuracy of the region of interest is represented by the display time of the bounding box, in response to detection of the region of interest in both the center region 43 and the peripheral region 44, for example, first, a bounding box 54 used to provide a notification of the region of interest detected in the center region 43 and a bounding box 55 used to provide a notification of the region of interest detected in the peripheral region 44 are displayed.

Then, at the point in time when a first display time elapses after the detection of the region of interest, the bounding box 54 continues to be displayed (indicated by a solid line), whereas the bounding box 55 is hidden (indicated by a broken line). After the elapse of the first display time, at the point in time when a second display time elapses, both the bounding boxes 54 and 55 are hidden. This allows the user to grasp the detection accuracy using the display time of the bounding boxes 54 and 55. That is, the user is able to grasp that the detection accuracy of the region of interest detected in the center region 43 is high, whereas the detection accuracy of the region of interest detected in the peripheral region 44 is low.

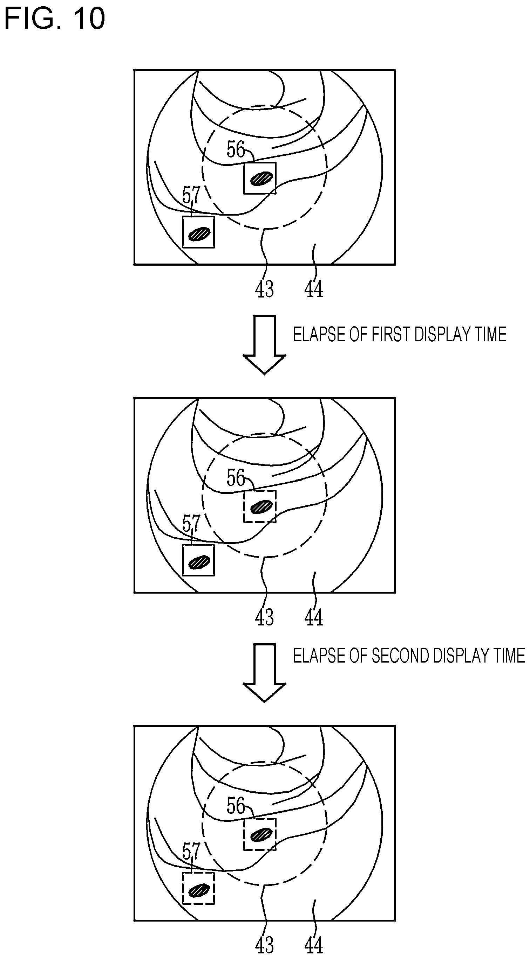

On the other hand, as illustrated in Fig. 10, when a bounding box is used as the specific geometric shape and the likelihood of oversight of the region of interest is represented by the display time of the bounding box, for example, first, a bounding box 56 used to provide a notification of the region of interest detected in the center region 43 and a bounding box 57 used to provide a notification of the region of interest detected in the peripheral region 44 are displayed. Then, at the point in time when a first display time elapses after the detection of the region of interest, the bounding box 57 continues to be displayed (indicated by a solid line), whereas the bounding box 56 is hidden (indicated by a broken line). This can prevent the region of interest detected in the peripheral region 44 from being overlooked since the bounding box 57 is displayed for a long time. In contrast, the region of interest detected in the center region 43 can be made less noticeable since the bounding box 56 is displayed for a short time.

Further, when the detection result of the region of interest is represented by a specific geometric shape, the transparency of the specific geometric shape used to provide a notification of the region of interest detected in the center region 43 is made different from the transparency of the specific geometric shape used to provide a notification of the region of interest detected in the peripheral region 44. The transparency refers to the degree to which a photographic subject image behind a specific geometric shape is visible in a medical image. If the transparency is high, the photographic subject image behind the specific geometric shape is see-through and visible. If the transparency is low, the photographic subject image behind the specific geometric shape is less visible due to the specific geometric shape.

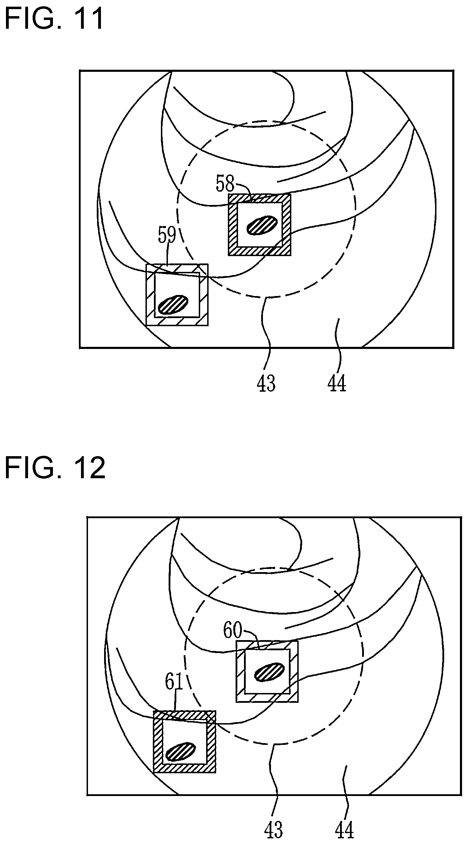

As illustrated in Fig. 11, when a bounding box is used as the specific shape and the detection accuracy of the region of interest is represented by the transparency of the bounding box, it is preferable that the transparency of a bounding box 58 used to provide a notification of the region of interest detected in the center region 43 be lower than the transparency of a bounding box 59 used to provide a notification of the region of interest detected in the peripheral region 44. This allows the user to grasp the detection accuracy using the transparency of the bounding boxes 58 and 59. In Fig. 11, the transparency is represented by the density of hatching such that the transparency decreases as the density increases and the transparency increases as the density decreases.

On the other hand, when a bounding box is used as the specific geometric shape and the likelihood of oversight of the region of interest is represented by the transparency of the bounding box, as illustrated in Fig. 12, it is preferable that the transparency of a bounding box 60 used to provide a notification of the region of interest detected in the center region 43 be higher than the transparency of a bounding box 61 used to provide a notification of the region of interest detected in the peripheral region 44. This can prevent the region of interest detected in the peripheral region 44 from being overlooked since the transparency of the bounding box 61 is low and the bounding box 61 is easily visually recognized. In contrast, the region of interest detected in the center region 43 can be made less noticeable since the transparency of the bounding box 60 is high and thus the visibility of the bounding box 60 is low.

Further, when the detection result of the region of interest is represented by a specific color region, the color of a specific color region used to provide a notification of the region of interest detected in the center region 43 is made different from the color of a specific color region used to provide a notification of the region of interest detected in the peripheral region 44. Preferably, each specific color region includes the region of interest. Further, preferably, a pseudo-color different from a color in the living body, such as red, is used for each specific color region. It is preferable to use a color distinguishable from the color of the region of interest.

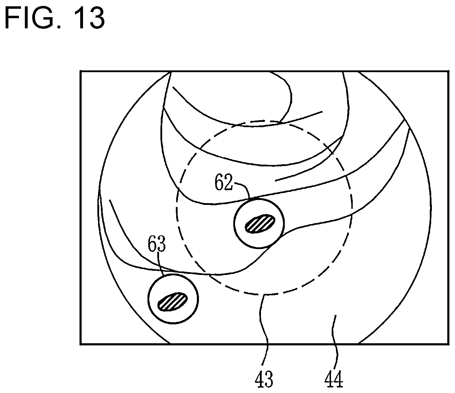

In this case, when the color of the specific color region is represented by the detection accuracy of the region of interest, as illustrated in Fig. 13, a specific color region 62 used to provide a notification of the region of interest detected in the center region 43 is displayed in a high-visibility color (for example, a color having high lightness and saturation), whereas a specific color region 63 used to provide a notification of the region of interest detected in the peripheral region 44 is displayed in a low-visibility color (for example, a color having low lightness and saturation). This allows the user to grasp the detection accuracy using the color of the specific color regions 62 and 63. That is, the user is able to grasp that the detection accuracy of the region of interest detected in the center region 43 is high, whereas the detection accuracy of the region of interest detected in the peripheral region 44 is low.

On the other hand, when the likelihood of oversight of the region of interest is represented by the color of a specific color region, although not illustrated, a specific color region used to provide a notification of the region of interest detected in the center region 43 is displayed in a low-visibility color (for example, a color having low lightness and saturation), whereas a specific color region used to provide a notification of the region of interest detected in the peripheral region 44 is displayed in a high-visibility color (for example, a color having high lightness and saturation). This can prevent the region of interest detected in the peripheral region 44 from being overlooked since the visibility of the specific color region is high. In contrast, the region of interest detected in the center region 43 can be made less noticeable since the visibility of the specific color region is low.

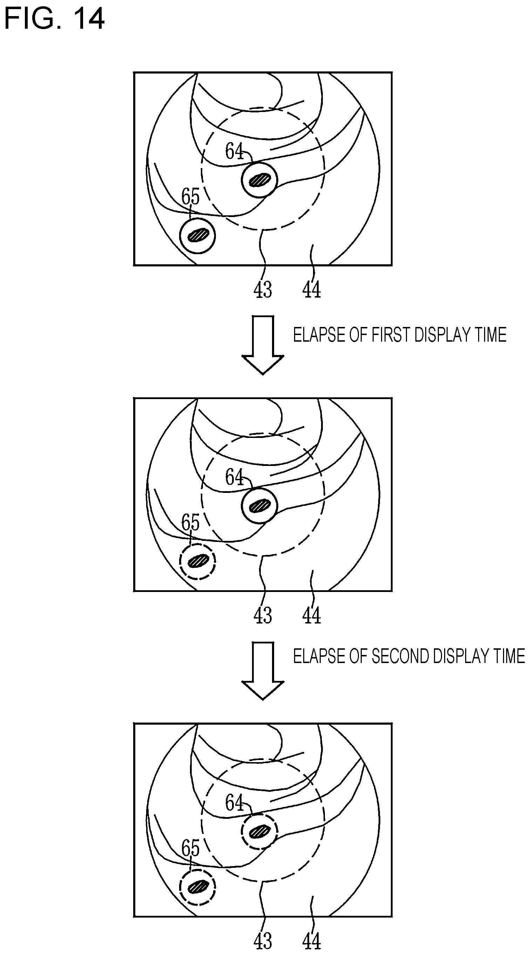

Further, when the detection result of the region of interest is represented by a specific color region such as a pseudo-color completely different from a color in the living body, the time (display time) during which a specific color region used to provide a notification of the region of interest detected in the center region 43 continues to be displayed on the monitor 34 is made different from the display time of a specific color region used to provide a notification of the region of interest detected in the peripheral region 44. In this case, when the detection accuracy of the region of interest is represented by the display time of a specific color region and the region of interest is detected in both the center region 43 and the peripheral region 44, as illustrated in Fig. 14, first, a specific color region 64 used to provide a notification of the region of interest detected in the center region 43 and a specific color region 65 used to provide a notification of the region of interest detected in the peripheral region 44 are displayed.

Then, at the point in time when a first display time elapses after the detection of the region of interest, the specific color region 64 continues to be displayed (indicated by a solid line), whereas the specific color region 65 is hidden (indicated by a broken line). The term "being hidden" means that there is no difference between the color of a specific color region and the color of any other region. After the elapse of the first display time, at the point in time when a second display time elapses, both the specific color regions 64 and 65 are hidden. This allows the user to grasp the detection accuracy using the length of the display time of the specific color regions 64 and 65. That is, the user is able to grasp that the detection accuracy of the region of interest detected in the center region 43 is high, whereas the detection accuracy of the region of interest detected in the peripheral region 44 is low.

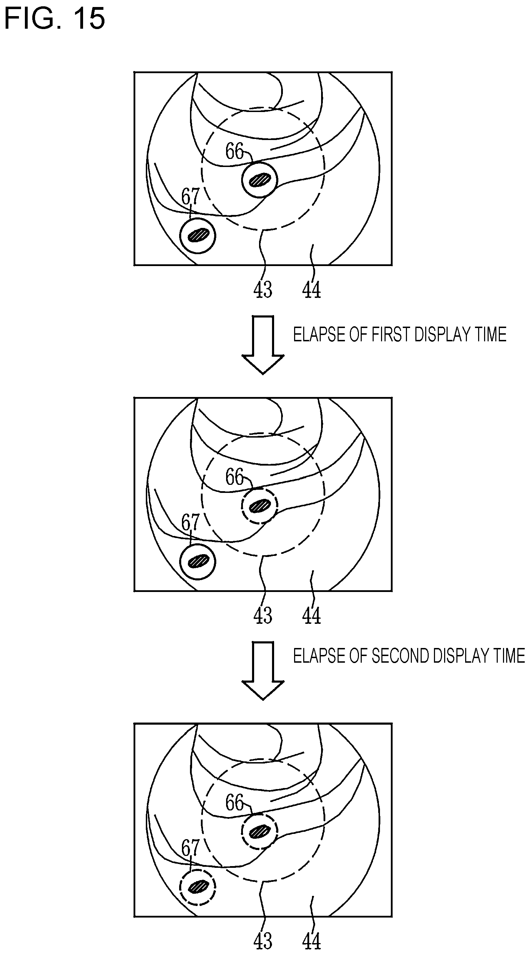

On the other hand, when the likelihood of oversight of the region of interest is represented by the display time of the specific color region and the region of interest is detected in both the center region 43 and the peripheral region 44, as illustrated in Fig. 15, first, a specific color region 66 used to provide a notification of the region of interest detected in the center region 43 and a specific color region 67 used to provide a notification of the region of interest detected in the peripheral region 44 are displayed. Then, at the point in time when a first display time elapses after the detection of the region of interest, the specific color region 67 continues to be displayed (indicated by a solid line), whereas the specific color region 66 is hidden (indicated by a broken line). This can prevent the region of interest detected in the peripheral region 44 from being overlooked since the specific color region 67 is displayed for a long time. In contrast, the region of interest detected in the center region 43 can be made less noticeable since the specific color region 66 is displayed for a short time.

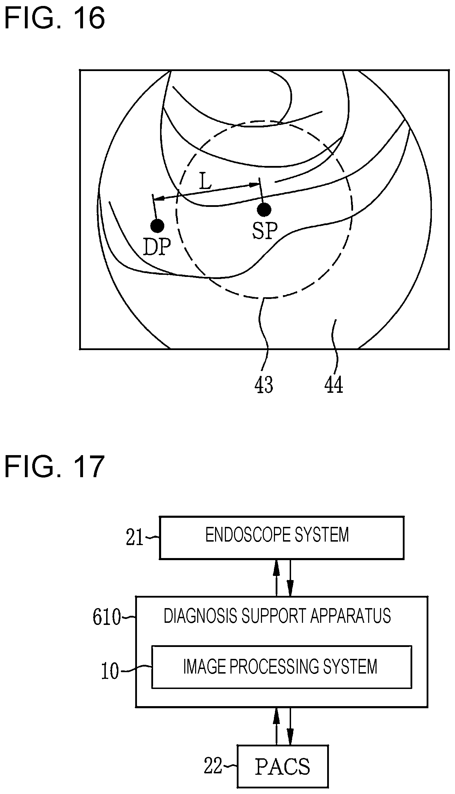

While the display style of the detection result of the region of interest is changed depending on whether the detection position of the region of interest is included in the center region or the peripheral region of the medical image, as illustrated in Fig. 16, the display style of the detection result of the region of interest is changed based on a specific distance L indicating the distance between a predetermined specific position SP and a detection position DP of the region of interest. The specific position SP is included in the center region 43 of the medical image. The display style of the detection result of the region of interest when the specific distance L falls within a specific range (inside-specific-range display style) is made different from the display style of the detection result of the region of interest when the specific distance L falls outside the specific range (outside-specific-range display style). Specifically, the inside-specific-range display style for the line thickness, size, display time, or transparency of a specific geometric shape, a specific color, or the display time of the specific color is made different from the outside-specific-range display style for the line thickness, size, display time, or transparency of a specific geometric shape, a specific color, or the display time of the specific color. A specific example for making this difference is similar to that described above.

Further, the medical image may be divided into two regions, namely, a center region or a peripheral region, and the display style of the detection result of the region of interest in either region may be changed. Alternatively, the medical image may be divided into three or more regions, and the display style of the detection result of the region of interest in each region may be changed. Further, while the medical image is divided concentrically into a center region and a peripheral region, the shape of divided portions may be any other shape such as a rectangular shape. Alternatively, the medical image may be divided into a plurality of types of portions such as concentric circles and rectangular shapes in combination.

While a bounding box is used as a specific geometric shape, any other geometric shape may be used. For example, a bounding box may be colored, or the observation target included in the region of interest may be indicated by an icon (a finger mark or a mouse cursor) or the like.

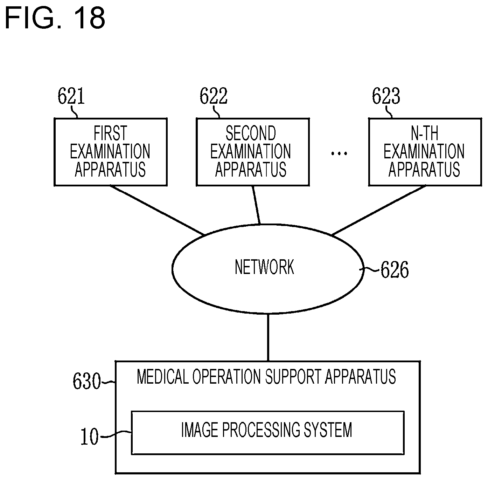

As illustrated in Fig. 17, a diagnosis support apparatus 610 to be used in combination with the endoscope system 21 or any other modality and the PACS 22 can include the image processing system 10 according to the embodiment described above and other modifications. As illustrated in Fig. 18, for example, a medical operation support apparatus 630 including the endoscope system 21 and to be connected to various examination apparatuses such as a first examination apparatus 621, a second examination apparatus 622, ..., and an N-th examination apparatus 623 via a desired network 626 can include the image processing system 10 according to the embodiment described above and other modifications.

Additionally, the image processing system 10, the endoscope system 21, and various apparatuses or systems including the image processing system 10 can be used with various modifications or the like described below.

As the medical image, a normal light image obtained by irradiation with light in the white range or, as light in the white range, light in a plurality of wavelength ranges.

When an image obtained by irradiation with light in a specific wavelength range is used as the medical image, the specific wavelength range may be a range narrower than the white wavelength range.

The specific wavelength range is, for example, the blue range or green range in the visible range.

When the specific wavelength range is the blue range or green range in the visible range, preferably, the specific wavelength range includes a wavelength range greater than or equal to 390 nm and less than or equal to 450 nm or greater than or equal to 530 nm and less than or equal to 550 nm, and light in the specific wavelength range has a peak wavelength in a wavelength range greater than or equal to 390 nm and less than or equal to 450 nm or greater than or equal to 530 nm and less than or equal to 550 nm.

The specific wavelength range is, for example, the red range in the visible range.

When the specific wavelength range is the red range in the visible range, preferably, the specific wavelength range includes a wavelength range greater than or equal to 585 nm and less than or equal to 615 nm or greater than or equal to 610 nm and less than or equal to 730 nm, and light in the specific wavelength range has a peak wavelength in a wavelength range greater than or equal to 585 nm and less than or equal to 615 nm or greater than or equal to 610 nm and less than or equal to 730 nm.

The specific wavelength range may include, for example, a wavelength range in which a light absorption coefficient is different between oxyhemoglobin and reduced hemoglobin, and light in the specific wavelength range may have a peak wavelength in a wavelength range in which a light absorption coefficient is different between oxyhemoglobin and reduced hemoglobin.

When the specific wavelength range includes a wavelength range in which a light absorption coefficient is different between oxyhemoglobin and reduced hemoglobin, and light in the specific wavelength range has a peak wavelength in a wavelength range in which a light absorption coefficient is different between oxyhemoglobin and reduced hemoglobin, preferably, the specific wavelength range includes a wavelength range of 400 ± 10 nm, 440 ± 10 nm, 470 ± 10 nm, or greater than or equal to 600 nm and less than or equal to 750 nm, and light in the specific wavelength range has a peak wavelength in a wavelength range of 400 ± 10 nm, 440 ± 10 nm, 470 ± 10 nm, or greater than or equal to 600 nm and less than or equal to 750 nm.

When the medical image is an in-vivo image obtained by imaging of the inside of a living body, the in-vivo image may have information on fluorescence emitted from a fluorescent substance in the living body.

As the fluorescence, fluorescence obtained by irradiation of the inside of a living body with excitation light having a peak wavelength greater than or equal to 390 nm and less than or equal to 470 nm may be used.

When the medical image is an in-vivo image obtained by imaging of the inside of a living body, the wavelength range of infrared light may be used as the specific wavelength range described above.

When the medical image is an in-vivo image obtained by imaging of the inside of a living body and the wavelength range of infrared light is used as the specific wavelength range described above, preferably, the specific wavelength range includes a wavelength range greater than or equal to 790 nm and less than or equal to 820 nm or greater than or equal to 905 nm and less than or equal to 970 nm, and light in the specific wavelength range has a peak wavelength in a wavelength range greater than or equal to 790 nm and less than or equal to 820 nm or greater than or equal to 905 nm and less than or equal to 970 nm.

The medical image acquisition unit 11 can have a special light image acquisition unit that acquires a special light image having a signal in the specific wavelength range on the basis of a normal light image obtained by irradiation with light in the white range or, as light in the white range, light in a plurality of wavelength ranges. In this case, the special light image can be used as the medical image.

The signal in the specific wavelength range can be obtained by calculation based on color information of RGB or CMY included in the normal light image.

A feature value image generation unit can be included that generates a feature value image by using calculation based on at least one of a normal light image obtained by irradiation with light in the white range or, as light in the white range, light in a plurality of wavelength ranges and a special light image obtained by irradiation with light in the specific wavelength range. In this case, the feature value image can be used as the medical image.

In the endoscope system 21, a capsule endoscope can be used as the endoscope 31. In this case, the light source device 32 and a portion of the processor device 33 can be mounted in the capsule endoscope.

In the embodiment described above and modifications, the hardware structure of processing units that execute various types of processing, such as the medical image acquisition unit 11, the medical image analysis processing unit 12, the display control unit 15, the input receiving unit 16, the overall control unit 17, the medical image acquisition unit 35, the medical image analysis processing unit 36, the display control unit 37, the region-of-interest detection unit 41, and the display style determination unit 42, is implemented as various processors described hereinbelow. The various processors include a CPU (Central Processing Unit), which is a general-purpose processor executing software (program) to function as various processing units, a programmable logic device (PLD) such as an FPGA (Field Programmable Gate Array), which is a processor whose circuit configuration is changeable after manufacture, a dedicated electric circuit, which is a processor having a circuit configuration specifically designed to execute various types of processing, a GPU (Graphical Processing Unit) that performs a large amount of processing such as image processing in parallel, and so on.

A single processing unit may be configured as one of the various processors or as a combination of two or more processors of the same type or different types (for example, a plurality of FPGAs, a combination of a CPU and an FPGA, or a combination of a CPU and a GPU). Alternatively, a plurality of processing units may be configured as a single processor. Examples of configuring a plurality of processing units as a single processor include, first, a form in which, as typified by a computer such as a client or a server, the single processor is configured as a combination of one or more CPUs and software and the processor functions as the plurality of processing units. The examples include, second, a form in which, as typified by a system on chip (SoC) or the like, a processor is used in which the functions of the entire system including the plurality of processing units are implemented as one IC (Integrated Circuit) chip. As described above, the various processing units are configured by using one or more of the various processors described above as a hardware structure.

More specifically, the hardware structure of these various processors is an electric circuit (circuitry) in which circuit elements such as semiconductor elements are combined. The hardware structure of the storage unit is a storage device such as an HDD (hard disc drive) or an SSD (solid state drive).

The invention is defined in the following claims. Other embodiments, examples, methods, items etc. are not a part of the invention and are disclosed for illustrative purposes only.

### Reference Signs List

- 10: image processing system
- 11: medical image acquisition unit
- 12: medical image analysis processing unit
- 13: display unit
- 15: display control unit
- 16: input receiving unit
- 17: overall control unit
- 18: storage unit
- 21: endoscope system
- 22: PACS
- 31: endoscope
- 32: light source device
- 33: processor device
- 34: monitor
- 35: medical image acquisition unit
- 36: medical image analysis processing unit
- 37: display control unit
- 41: region-of-interest detection unit
- 42: display style determination unit
- 43: center region
- 44: peripheral region
- 46: bounding box
- 46 to 61: bounding box
- 62 to 67: specific color region
- 610: diagnosis support apparatus
- 621: first examination apparatus
- 622: second examination apparatus
- 623: N-th examination apparatus
- 626: network
- 630: medical operation support apparatus
- SP: specific position
- DP: detection position
- L: specific distance

## Claims

1. A medical image processing system comprising:
a medical image acquisition unit that acquires a medical image obtained by imaging of an observation target by using an endoscope system;
a region-of-interest detection unit that detects a region of interest from the medical image; and
a display control unit that displays a detection result of the region of interest on a display unit using a display style that differs depending on at least a detection position of the region of interest which is included in the medical image,
wherein a display style of the detection result of the region of interest when a specific distance indicating a distance in the medical image between a predetermined specific position and the detection position of the region of interest falls within a specific range is different from a display style of the detection result of the region of interest when the specific distance falls outside the specific range, and
**characterised in that**
the specific position is included in a center region of the medical image.

2. The medical image processing system according to claim 1, wherein when the detection result of the region of interest is represented by a specific geometric shape, a line thickness of the specific geometric shape differs depending on the detection position of the region of interest.

3. The medical image processing system according to claim 1 or 2, wherein when the detection result of the region of interest is represented by a specific geometric shape, a size of the specific geometric shape differs depending on the detection position of the region of interest.

4. The medical image processing system according to claim 1 or 2, wherein when the detection result of the region of interest is represented by a specific geometric shape, a display time of the specific geometric shape differs depending on the detection position of the region of interest.

5. The medical image processing system according to any one of claims 1 to 4, wherein when the detection result of the region of interest is represented by a specific geometric shape, a transparency of the specific geometric shape differs depending on the detection position of the region of interest.

6. The medical image processing system according to any one of claims 1 to 4, wherein when the detection result of the region of interest is represented by a specific color region, a color of the specific color region differs depending on the detection position of the region of interest.

7. The medical image processing system according to claim 1, wherein when the detection result of the region of interest is represented by a specific color region, a display time of the specific color region differs depending on the detection position of the region of interest.

## Patentansprüche

1. Verarbeitungssystem für medizinische Bilder, umfassend:
eine Erfassungseinheit für medizinische Bilder, die ein medizinisches Bild, das durch Abbilden eines Beobachtungsziels durch Verwenden eines Endoskopsystems erhalten wird, erfasst;
eine Detektionseinheit für Bereich von Interesse, die einen Bereich von Interesse aus dem medizinischen Bild detektiert; und
eine Anzeigesteuereinheit, die ein Detektionsergebnis des Bereichs von Interesse auf einer Anzeigeeinheit unter Verwendung eines Anzeigestils anzeigt, der sich in Abhängigkeit von mindestens einer Detektionsposition des Bereichs von Interesse, der in dem medizinischen Bild enthalten ist, unterscheidet,
wobei ein Anzeigestil des Detektionsergebnisses des Bereichs von Interesse, wenn eine spezifische Distanz, die eine Distanz in dem medizinischen Bild zwischen einer vorbestimmten spezifischen Position und der Detektionsposition des Bereichs von Interesse angibt, innerhalb eines spezifischen Bereichs fällt, sich von einem Anzeigestil des Detektionsergebnisses des Bereichs von Interesse, wenn die spezifische Distanz außerhalb des spezifischen Bereichs fällt, unterscheidet, und
**dadurch gekennzeichnet, dass**
die spezifische Position in einem mittleren Bereich des medizinischen Bildes enthalten ist.

2. Verarbeitungssystem für medizinische Bilder nach Anspruch 1, wobei, wenn das Detektionsergebnis des Bereichs von Interesse durch eine spezifische geometrische Form dargestellt wird, eine Liniendicke der spezifischen geometrischen Form in Abhängigkeit von der Detektionsposition des Bereichs von Interesse unterschiedlich ist.

3. Verarbeitungssystem für medizinische Bilder nach Anspruch 1 oder 2, wobei, wenn das Detektionsergebnis des Bereichs von Interesse durch eine spezifische geometrische Form dargestellt wird, eine Größe der spezifischen geometrischen Form in Abhängigkeit von der Detektionsposition des Bereichs von Interesse unterschiedlich ist.

4. Verarbeitungssystem für medizinische Bilder nach Anspruch 1 oder 2, wobei, wenn das Detektionsergebnis des Bereichs von Interesse durch eine spezifische geometrische Form dargestellt wird, eine Anzeigezeit der spezifischen geometrischen Form in Abhängigkeit von der Detektionsposition des Bereichs von Interesse unterschiedlich ist.

5. Verarbeitungssystem für medizinische Bilder nach einem der Ansprüche 1 bis 4, wobei, wenn das Detektionsergebnis des Bereichs von Interesse durch eine spezifische geometrische Form dargestellt wird, eine Transparenz der spezifischen geometrischen Form in Abhängigkeit von der Detektionsposition des Bereichs von Interesse unterschiedlich ist.

6. Verarbeitungssystem für medizinische Bilder nach einem der Ansprüche 1 bis 4, wobei, wenn das Detektionsergebnis des Bereichs von Interesse durch ein Bereich spezifischer Farbe dargestellt wird, eine Farbe des Bereichs spezifischer Farbe in Abhängigkeit von der Detektionsposition des Bereichs von Interesse unterschiedlich ist.

7. Verarbeitungssystem für medizinische Bilder nach Anspruch 1, wobei, wenn das Detektionsergebnis des Bereichs von Interesse durch ein Bereich spezifischer Farbe dargestellt wird, eine Anzeigezeit des Bereichs spezifischer Farbe in Abhängigkeit von der Detektionsposition des Bereichs von Interesse unterschiedlich ist.

## Revendications

1. Système de traitement d'images médicales comprenant :
une unité d'acquisition d'images médicales qui acquiert une image médicale obtenue par imagerie d'une cible d'observation en utilisant un système d'endoscope ;
une unité de détection de région d'intérêt qui détecte une région d'intérêt à partir de l'image médicale ; et
une unité de contrôle d'affichage qui affiche un résultat de détection de la région d'intérêt sur une unité d'affichage à l'aide d'un style d'affichage qui diffère selon au moins une position de détection de la région d'intérêt qui est incluse dans l'image médicale,
dans lequel un style d'affichage du résultat de détection de la région d'intérêt lorsqu'une distance spécifique indiquant une distance dans l'image médicale entre une position spécifique prédéterminée et la position de détection de la région d'intérêt tombe dans une plage spécifique est différente d'un style d'affichage du résultat de détection de la région d'intérêt lorsque la distance spécifique tombe en dehors de la plage spécifique, et
**caractérisé en ce que**
la position spécifique est incluse dans une région centrale de l'image médicale.

2. Système de traitement d'images médicales selon la revendication 1, dans lequel lorsque le résultat de détection de la région d'intérêt est représenté par une forme géométrique spécifique, une épaisseur de ligne de la forme géométrique spécifique diffère selon la position de détection de la région d'intérêt.

3. Système de traitement d'images médicales selon la revendication 1 ou la revendication 2, dans lequel lorsque le résultat de détection de la région d'intérêt est représenté par une forme géométrique spécifique, une taille de la forme géométrique spécifique diffère selon la position de détection de la région d'intérêt.

4. Système de traitement d'images médicales selon la revendication 1 ou la revendication 2, dans lequel lorsque le résultat de détection de la région d'intérêt est représenté par une forme géométrique spécifique, un temps d'affichage de la forme géométrique spécifique diffère selon la position de détection de la région d'intérêt.

5. Système de traitement d'images médicales selon l'une quelconque des revendications 1 à 4, dans lequel lorsque le résultat de détection de la région d'intérêt est représenté par une forme géométrique spécifique, une transparence de la forme géométrique spécifique diffère selon la position de détection de la région d'intérêt.

6. Système de traitement d'images médicales selon l'une quelconque des revendications 1 à 4, dans lequel lorsque le résultat de détection de la région d'intérêt est représenté par une région de couleur, une couleur de la région de couleur spécifique diffère selon la position de détection de la région d'intérêt.

7. Système de traitement d'images médicales selon la revendication 1, dans lequel lorsque le résultat de détection de la région d'intérêt est représenté par une région de couleur, un temps d'affichage de la région de couleur spécifique diffère selon la position de détection de la région d'intérêt.
